(19) Europäisches Patentamt
European Patent Office
Office européen des brevets

(11) EP 4 729 928 A1

(12) EUROPEAN PATENT APPLICATION
published in accordance with Art. 153(4) EPC

(43) Date of publication:
22.04.2026 Bulletin 2026/17

(21) Application number: 24843531.5

(22) Date of filing: 18.07.2024

(51) International Patent Classification (IPC):
G01N 21/65 (2006.01)    G01J 3/44 (2006.01)
H01M 10/42 (2006.01)

(52) Cooperative Patent Classification (CPC):
G01N 21/65; G01J 3/44; G16C 20/20; H01M 10/42;
B82Y 35/00; G01N 2021/0118; G01N 2201/1296

(86) International application number:
PCT/KR2024/010389

(87) International publication number:
WO 2025/018817 (23.01.2025 Gazette 2025/04)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH MA MD TN

(30) Priority: 18.07.2023 KR 20230093391

(71) Applicant: LG Chem, Ltd.
Seoul 07336 (KR)

(72) Inventors:
• KONG, Chang Sun
Daejeon 34122 (KR)
• LEE, Kyu Hwang
Daejeon 34122 (KR)

(74) Representative: Hoffmann Eitle
Patent- und Rechtsanwälte PartmbB
Arabellastraße 30
81925 München (DE)

(54) **DIAGNOSTIC DEVICE AND METHOD FOR OPERATING DIAGNOSTIC DEVICE**

(57) A diagnosis apparatus according to an embodiment disclosed herein includes a communication circuit configured to obtain, from a Raman spectroscope, information about a Raman spectrum for a mixed sample of different carbon materials including a carbon nanotube (CNT) and a processor configured to obtain intensities of nodes divided by reference wavenumber spacings of a spectrum, based on the information, generate a network of the nodes based on the intensities, and calculate a CNT content of the sample based on characteristics of the network.

FIG.1

EP 4 729 928 A1

## Description

## TECHNICAL FIELD

CROSS-REFERENCE TO RELATED APPLICATION

This application claims priority to and the benefit of Korean Patent Application No. 10-2023-0093391 filed in the Korean Intellectual Property Office on July 18, 2023, the entire contents of which are incorporated herein by reference.

TECHNICAL FIELD

Embodiments disclosed herein relate to a diagnosis apparatus and an operating method thereof.

## BACKGROUND ART

A carbon nanotube (CNT), which is an allotrope of carbon in which a hexagonal honeycomb-shaped graphene sheet having one carbon atom bonded to three other carbon atoms is rolled up into a nano-sized diameter, may be manufactured to have a diameter of 1 nm to several tens of nm and various lengths of several $\mu$m to several tens of nm, thus having a structural feature of a very large aspect ratio.

The CNT is generally classified into a single wall nanotube and a multi-walled nanotube, and is a large carbon molecule having various physical and chemical properties according to a size, a shape, a functional group of a surface, coating, doping, etc. The CNT is lightweight, has excellent electrical and thermal conductivity, and has a tensile equivalent to steel.

For example, when the CNT is used mixed with an electrode material of a secondary battery, battery performance may be significantly improved. By using the single wall or multi-walled CNT in a mixture thereof as a component of a cathode or anode, numerous methods for improving electrical conductivity, mechanical physical properties, and lifetime of an electrode have been developed.

When different types of carbon materials such as graphite, carbon black (CB), and a CNT are used as a mixture of two or more of them in a cathode or anode of a lithium-ion battery, there is a need for a method for quantitatively and accurately diagnosing whether the content of the CNT is uniformly distributed in the electrode and how the content of the CNT and the distribution of the CNT in the electrode change during use of the battery.

DISCLOSURE

TECHNICAL PROBLEM

Embodiments disclosed herein aim to quantitatively obtain the content of the CNT from Raman spectrum data measured for a mixed sample including a plurality of different carbon materials including the CNT.

Technical problems of the embodiments disclosed herein are not limited to the above-described technical problems, and other unmentioned technical problems would be clearly understood by one of ordinary skill in the art from the following description.

TECHNICAL SOLUTION

A diagnosis apparatus according to an embodiment disclosed herein includes a communication circuit configured to obtain, from a Raman spectroscope, information about a Raman spectrum for a mixed sample of different carbon materials including a carbon nanotube (CNT) and a processor configured to obtain intensities of nodes divided by reference wavenumber spacings of a spectrum, based on the information, generate a network of the nodes based on the intensities, and calculate a CNT content of the sample based on characteristics of the network.

An operating method of a diagnosis apparatus according to an embodiment disclosed herein includes obtaining, from a Raman spectroscope, information about a Raman spectrum for a sample, obtaining intensities of nodes divided by reference wavenumber spacings, based on the information, generate a network of the nodes based on the intensities, and calculating a CNT content of the sample based on characteristics of the network.

A battery management apparatus according to an embodiment disclosed herein includes a communication unit configured to obtain, from a Raman spectroscope, information about a Raman spectrum for a mixed sample of different carbon materials including a CNT and a controller configured to obtain intensities of nodes divided by reference wavenumber spacings of a spectrum, based on the information, generate a network of the nodes based on the intensities, and calculate a CNT content of the sample based on characteristics of the network.

ADVANTAGEOUS EFFECTS

The diagnosis apparatus and the operating method thereof according to various embodiments disclosed herein may obtain content information of the CNT from a Raman spectrum of a plurality of carbon mixed samples including the CNT.

The effects of the diagnosis apparatus and the operating method thereof according to the disclosure of the present document are not limited to the effects mentioned above, and other effects not mentioned will be clearly understood by those of ordinary skill in the art according to the disclosure of the present document.

## DESCRIPTION OF DRAWINGS

FIG. 1 is a block diagram illustrating a configuration of a diagnosis apparatus according to an embodiment disclosed herein.

FIG. 2 illustrates a Raman spectrum obtained from different standard samples, according to an embodiment disclosed herein.

FIG. 3 is a graph analyzing a Raman spectrum of a random standard sample, according to an embodiment disclosed herein.

FIG. 4 illustrates an adjacency matrix and a distance matrix for a network architecture that converts Raman spectrum data, according to an embodiment disclosed herein.

FIG. 5 illustrates changes in network entropy and network energy with respect to changes in CNT content, according to an embodiment disclosed herein.

FIG. 6 illustrates a correlation graph between an actual CNT content of standard samples and a CNT content calculated from network feature values converted from a Raman spectrum, according to an embodiment disclosed herein.

FIG. 7 is a flowchart of an operating method of a diagnosis apparatus according to an embodiment disclosed herein.

FIG. 8 illustrates a battery pack according to an embodiment disclosed herein.

FIG. 9 is a block diagram illustrating a configuration of a battery management apparatus, according to an embodiment disclosed herein.

With regard to the description of the drawings, similar reference numerals may be used to refer to similar or related components.

## MODE FOR INVENTION

Hereinafter, embodiments of the present disclosure will be described with reference to the accompanying drawings. However, the description is not intended to limit the present disclosure to particular embodiments, and it should be construed as including various modifications, equivalents, and/or alternatives according to the embodiments of the present disclosure.

It should be appreciated that embodiments of the present document and the terms used therein are not intended to limit the technological features set forth herein to a particular embodiment and include various changes, equivalents, or replacements for a corresponding embodiment. With regard to the description of the drawings, similar reference numerals may be used to refer to similar or related elements. It is to be understood that a singular form of a noun corresponding to an item may include one or more of the things, unless the relevant context clearly indicates otherwise.

As used herein, each of such phrases as "A or B," "at least one of A and B," "at least one of A or B," "A, B, or C," "at least one of A, B, and C," and "at least one of A, B, or C," may include any one of, or all possible combinations of the items enumerated together in a corresponding one of the phrases. Such terms as "1st", "2nd," "first", "second", "A", "B", "(a)", or "(b)" may be used to simply distinguish a corresponding component from another, and does not limit the components in other aspect (e.g., importance or order), unless mentioned otherwise.

Herein, it is to be understood that when an element (e.g., a first element) is referred to, with or without the term "operatively" or "communicatively", as "connected with", "coupled with", or "linked with", or "coupled to" or "connected to" to another element (e.g., a second element), it means that the element may be connected with the other element directly (e.g., wiredly or wirelessly), or indirectly (e.g., via a third element).

A method according to various embodiments disclosed herein may be included and provided in a computer program product. The computer program product may be traded as a product between a seller and a buyer. The computer program product may be distributed in the form of a machine-readable storage medium (e.g., compact disc read only memory (CD-ROM)), or be distributed (e.g., downloaded or uploaded) online via an application store, or between two user devices directly. If distributed online, at least part of the computer program product may be temporarily generated or at least temporarily stored in the machine-readable storage medium, such as memory of the manufacturer's server, a server of the application store, or a relay server.

According to embodiments disclosed herein, each component (e.g., a module or a program) of the above-described components may include a single entity or multiple entities, and some of the multiple entities may be separately disposed in different components. According to various embodiments disclosed herein, one or more of the above-described components may be omitted, or one or more other components may be added. Alternatively or additionally, a plurality of components (e.g., modules or programs) may be integrated into a single component. In such a case, according to various embodiments, the integrated component may still perform one or more functions of each of the plurality of components in the same or similar manner as they are performed by a corresponding one of the plurality of components before the integration. According to embodiments disclosed herein, operations performed by the module, the program, or another component may be carried out sequentially, in parallel, repeatedly, or heuristically, or one or more of the operations may be executed in a different order or omitted, or one or more other operations may be added.

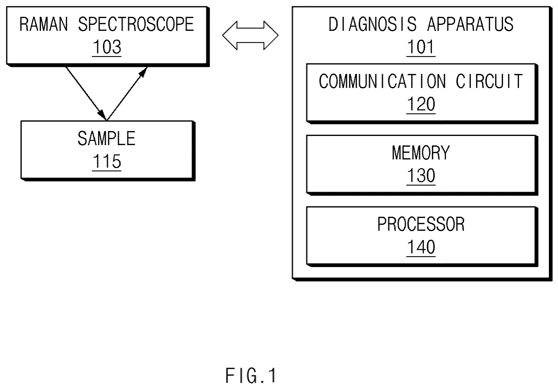

FIG. 1 is a block diagram illustrating a configuration of a diagnosis apparatus according to an embodiment disclosed herein.

Referring to FIG. 1, a diagnosis apparatus 101 may be wiredly and/or wirelessly connected to a Raman spectroscope 103.

According to an embodiment, connection between the diagnosis apparatus 101 and the Raman spectroscope 103 may be communication connection through a wired and/or wireless network. According to an embodiment, the wired network may be based on a local area network (LAN) communication or a power-line communication. According to an embodiment, the wireless network may be based on a short-range communication network (e.g., Bluetooth, Wireless Fidelity (WiFi), or Infrared Data Association (IrDA)) or a remote-range communication network (e.g., a cellular network, a 4th-Generation (4G) network, a 5th-Generation (5G) network).

According to another embodiment, the connection between the diagnosis apparatus 101 and the Raman spectroscope 103 may be connection using a device-to-device communication scheme (e.g., a bus, a general-purpose input and output (GPIO), a serial peripheral interface (SPI), or a mobile industry processor interface (MIPI)).

According to an embodiment, the Raman spectroscope 103 may be a device that performs Raman spectroscopy. According to an embodiment, the Raman spectroscope 103 may be a device for identifying a structure, a type, a content, etc., of a molecule included in a sample 115 by using the Raman effect. According to an embodiment, the Raman spectroscope 103 may identify the structure, the type, the content, etc., of the molecule included in the sample 115 based on a principle that energy of laser irradiated to the sample 115 is absorbed by a difference in energy level of electrons of the molecule of the sample 115.

According to an embodiment, the diagnosis apparatus 101 may be a mobile device (e.g., a mobile phone, a laptop computer, a smart phone, a smart pad) or a computer (e.g., a general-purpose computer and a special-purpose computer).

According to an embodiment, the diagnosis apparatus 101 may include a communication circuit 120, a memory 130, and a processor 140. According to an embodiment, the diagnosis apparatus 101 shown in FIG. 1 may further include at least one component (e.g., a display, an input device, or an output device) in addition to components shown in FIG. 1.

According to an embodiment, the communication circuit 120 may establish a wired communication channel and/or a wireless communication channel between the diagnosis apparatus 101 and the Raman spectroscope 103, and transmit and receive data to and from the Raman spectroscope 103 through the established communication channel.

According to an embodiment, the memory 130 may include a volatile and/or a nonvolatile memory.

According to an embodiment, the memory 130 may store data used by at least one component (e.g., the processor 140) of the diagnosis apparatus 101. For example, the data may include a program (or an instruction related thereto), input data, or output data. According to an embodiment, the instruction, when executed by the processor 140, may cause the diagnosis apparatus 101 to perform operations defined by the instruction.

According to an embodiment, the processor 140 may include a central processing unit, an application processor, a graphic processing unit, a neural processing unit (NPU), an image signal processor, a sensor hub processor, or a communication processor.

According to an embodiment, the processor 140 may execute a program (or an instruction related thereto) to control at least one other component (e.g., a hardware or software component) of the diagnosis apparatus 101 connected to the processor 140 and may process or compute various data.

In an embodiment, the processor 140 and the diagnosis apparatus 101 connected to the processor 140 may be used as components included in a battery management system (BMS).

In an embodiment, the "BMS" including the processor 140 and the diagnosis apparatus 101 connected to the processor 140 may mean an interface of any type including the BMS.

In an embodiment of the present disclosure, a mobile device including a BMS apparatus according to the present application may be provided.

Herein, the "mobile device" may mean a device which is capable of moving or which may be easily carried by a user, and examples thereof may include electric vehicles, mobile devices, etc.

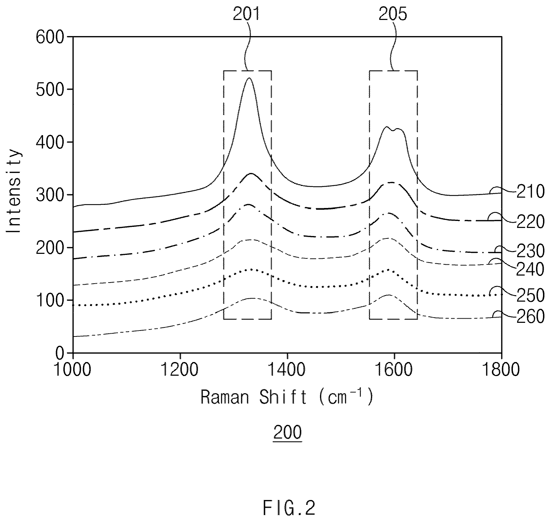

Hereinbelow, referring to FIGS. 2 to 6, a detailed description will be made of a method, performed by the diagnosis apparatus 101, of quantitatively measuring a content of a CNT included in the sample 115 based on spectrum data obtained from the Raman spectroscope 103.

Obtaining Equation

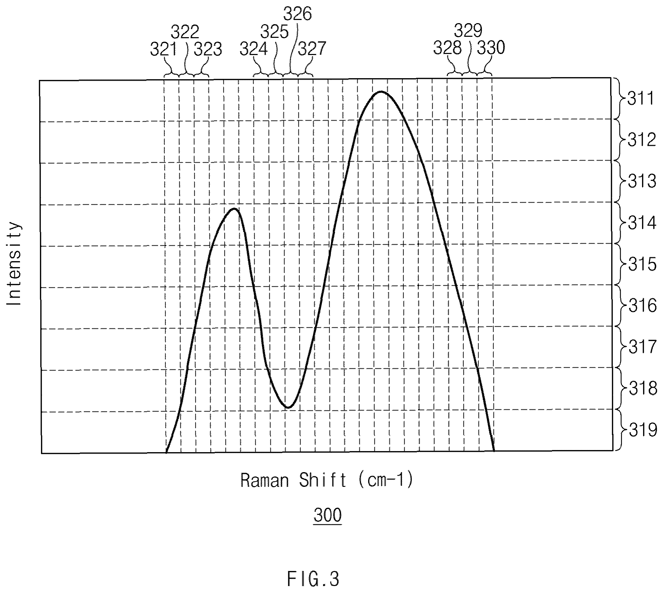

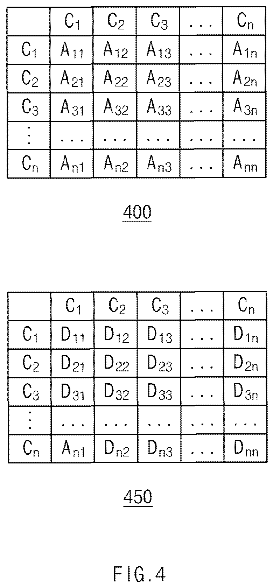

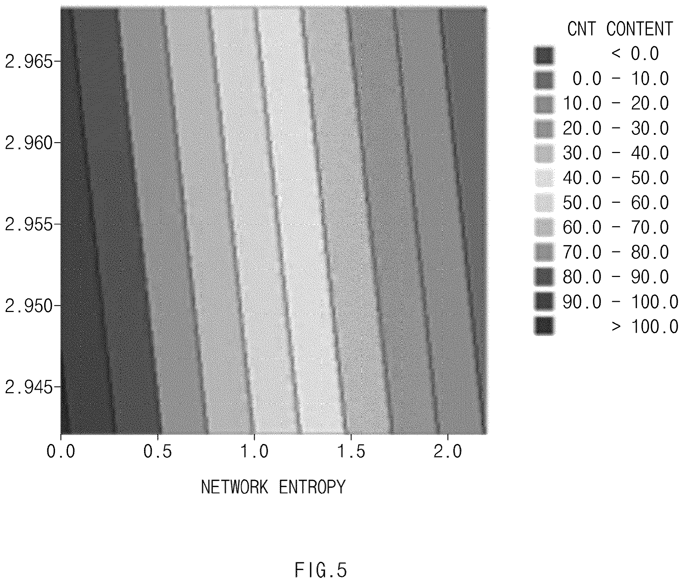

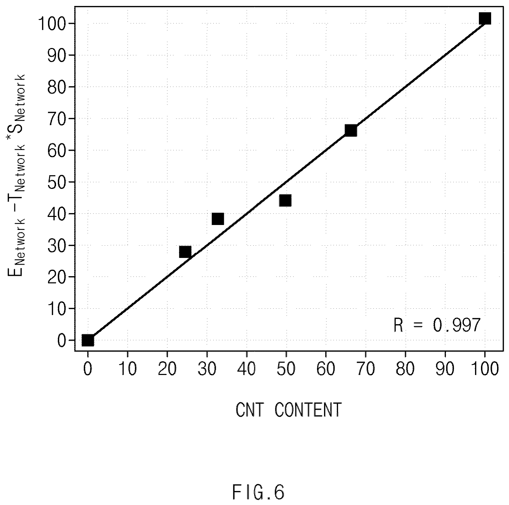

FIG. 2 illustrates a Raman spectrum obtained from standard samples having different compositions, according to an embodiment disclosed herein. FIG. 3 illustrates that a Raman spectrum of standard samples for converting peak strength information of a Raman spectrum into a network architecture is divided with respect to sections of an intensity of a unit spectrum and a reference wavenumber. FIG. 4 illustrates an adjacency matrix and a distance matrix that feature a network architecture, according to an embodiment disclosed herein. FIG. 5 illustrates changes in network entropy and network energy with respect to a CNT content, according to an embodiment disclosed herein. FIG. 6 illustrates a correlation graph between an actual CNT content of standard samples and a CNT content calculated from network feature values converted from a Raman spectrum, according to an embodiment disclosed herein.

The Raman spectroscope 103 may obtain Raman spectra 210, 220, 230, 240, 250, and 260 from different standard samples 115. According to an embodiment, the Raman spectroscope 103 may obtain the Raman spectra 210, 220, 230, 240, 250, and 260 in a wavenumber (Raman shift) from 1000 to 1800 $cm^{-1}$.

The different standard samples 115 may be samples in which the CNT is mixed at different mass ratios (weight percent, wt%). For example, CNT contents of the different standard samples 115 may be respectively 0, 25, 33.3, 50, 66,7, and 100 wt%. For example, the Raman spectra 210, 220, 230, 240, 250, and 260 may be obtained when the CNT contents of the standard samples 115 are 100, 66.7, 50, 33.3, 25, and 0 wt%, respectively.

The Raman spectra 210, 220, 230, 240, 250, and 260 of the different standard samples 115 may have peak values in a D band 201 around a wavelength of 1350 $cm^{-1}$ and a G band 205 around a wavelength of 1580 $cm^{-1}$.

The Raman spectroscope 103 may transmit information about the obtained Raman spectra 210, 220, 230, 240, 250, and 260 of the standard samples 115 having different compositions to the diagnosis apparatus 101.

The diagnosis apparatus 101 may obtain information about the Raman spectra 210, 220, 230, 240, 250, and 260 from the Raman spectroscope 103 through the communication circuit 120.

The processor 140 of the diagnosis apparatus 101 may analyze the Raman spectra 210, 220, 230, 240, 250, and 260. According to an embodiment, the processor 140 may divide a Raman spectrum at reference frequency spacings. The Raman spectrum divided by wavenumber ranges 321 to 330 may be referred to as a unit Raman spectrum.

According to an embodiment, the processor 140 may set an intensity of the unit Raman spectrum. Herein, the intensity of the unit Raman spectrum may be set by an intensity of a start point, an intensity of a midpoint, an intensity of the last point, or an average intensity for each section of a wavenumber range.

The processor 140 may classify unit Raman spectra as nodes in intensity ranges 311 to 319 for respective reference wavenumber sections, based on intensities of unit Raman spectra.

Referring to the graph 300 of FIG. 3, it may be seen that a Raman spectrum is subdivided into unit Raman spectra based on a grid of a unit intensity and a unit wavenumber spacing.

According to an embodiment, the processor 140 may generate an adjacency matrix 400 and a distance matrix 450 based on unit Raman spectra. Referring to FIG. 4, elements $A_{11}$, $A_{12}$, ..., $A_{1n}$, $A_{21}$, ..., $A_{nn}$ of the adjacency matrix 400 may indicate adjacencies between unit spectra $C_1$, $C_2$, ..., $C_n$, and elements $D_{11}$, $D_{12}$, ..., $D_{1n}$, $D_{21}$, ..., $D_{nn}$ of the distance matrix 450 may indicate distances between the unit spectra $C_1$, $C_2$, ..., $C_n$.

According to an embodiment, unit Raman spectra divided by the wavenumber ranges 321 to 330 may be understood as the unit spectra $C_1$, $C_2$, ..., $C_n$ of the adjacency matrix 400 and the distance matrix 450. For example, a unit Raman spectrum included in the wavenumber range 321 may be understood as a first node $C_1$, a unit Raman spectrum included in the wavenumber range 322 may be understood as a second node $C_2$, and a unit Raman spectrum included in the wavenumber range 323 may be understood as a third node $C_3$.

According to an embodiment, in such a way that unit spectra are set to be adjacent to each other (i.e., their elements have a value of 1) between adjacent intensity ranges and unit spectra are set not to be adjacent to each other (i.e., their elements have a value of 0) between non-adjacent intensity ranges, values of the elements $A_{11}$, $A_{12}$, ..., $A_{1n}$, $A_{21}$, ..., $A_{nn}$ of the adjacency matrix 400 may be assigned. For example, when the node $C_1$ corresponding to the wavenumber range 321 is included in the intensity range 319 and the node $C_2$ corresponding to the wavenumber range 322 is included in the intensity range 318, the elements $A_{12}$ and $A_{21}$ defined by the unit spectra $C_1$ and $C_2$ may have values of 1. In another example, when the node $C_1$ corresponding to the wavenumber range 321 is included in the intensity range 319 and the node $C_3$ corresponding to the wavenumber range 323 is included in the intensity range 316, the elements $A_{12}$ and $A_{13}$ defined by the unit spectra $C_1$ and $C_3$ may have values of 0. A product of assigned 0 or 1 and a weight factor may be used as a value of the elements of the adjacency matrix 400.

According to an embodiment, elements $D_{11}$, $D_{12}$, ..., $D_{1n}$, $D_{21}$, ..., $D_{nn}$ of the distance matrix 450 may be set as distances between the nodes $C_1$, $C_2$, ..., $C_n$ defined based on a unit intensity section and a unit wavenumber section. For example, a distance between unit spectra included in the same intensity range may be a value of at least 0 according to a difference between unit wavenumber sections. In another example, a distance between nodes included in different intensity ranges may be a value of at least 1 according to a difference between unit wavenumber sections. For example, a distance between a node included in the intensity range 319 and a node included in the intensity range 317 may be 2 in the wavenumber range 321 and the wavenumber range 322, but may be 11 in the wavenumber range 321 and the wavenumber range 324. A product of a distance between two different nodes and a preset weight factor may be used as a value of the elements of the distance matrix 450.

According to an embodiment, the processor 140 may obtain network characteristics of the nodes $C_1$, $C_2$, ..., $C_n$, based on the adjacency matrix 400 and the distance matrix 450. Herein, a network of the nodes $C_1$, $C_2$, ..., $C_n$ may indicate a connection structure (a distance and adjacency) between the nodes $C_1$, $C_2$, ..., $C_n$. The network characteristics may include a network energy $E_{network}$, a network entropy $S_{network}$, and a network temperature $T_{network}$.

According to an embodiment, the network energy $E_{network}$ may be expressed as a product of the number of nodes included in the network and a function of a distance between the nodes. For example, the network energy $E_{network}$ may be defined by Equation 1 below.

[Equation 1]

$$S_{network,i,j} = a * f(d_{i,j})$$

Herein, a may indicate number information of nodes, and $f(d_{i,j})$ may indicate a function of a distance d between $i^{th}$ and $j^{th}$ nodes. Herein, the function f may be a polynomial function set by a linear or non-linear regression technique. For example, the network energy $E_{network}$ may be a sum of network energies $E_{network,i,j}$ obtained for all nodes.

According to an embodiment, the network entropy $S_{network}$ may be expressed as a product of a function value for a reciprocal of a topological distance between two nodes and a logarithm of the function value for the reciprocal of the topological distance between the two nodes (herein, H may be $g(1/d_{ij})$, and $d_{ij}$ may be defined as a topological distance between the two nodes i and j). For example, the network entropy $S_{network}$ may be defined based on Equation 2.

[Equation 2]

$$S_{network} = H * \log H$$

Herein, log may mean a logarithmic function. Herein, H may be defined by Equation 3.

[Equation 3]

$$H = g(1/d_{i,j})$$

$g(1/d_{i,j})$ may indicate a function of a reciprocal of the distance d between the $i^{th}$ and $j^{th}$ nodes. Herein, the function g may be a polynomial function set by a linear or non-linear regression technique. For example, the network entropy $S_{network}$ may be a sum of network entropies $S_{network,i,j}$ obtained for all the nodes.

According to an embodiment, the network temperature $T_{network}$ may be set in advance (e.g., 45.87) according to a content of a CNT included in a standard sample.

According to an embodiment, the processor 140 may obtain information about a relationship between the network energy $E_{network}$, the network entropy $S_{network}$, and a CNT content. According to an embodiment, the processor 140 may obtain the information about the relationship between the network energy $E_{network}$, the network entropy $S_{network}$, and the CNT content, based on multiple linear regression (MLR). According to an embodiment, the information about the relationship may be obtained by a machine learning algorithm such as a support vector machine (SVM), a decision tree, a gradient boosting machine (GBM), artificial neural networks, etc., but the present disclosure is not limited to stated algorithms. According to an embodiment, the processor 140 may obtain the information about the relationship between the network energy $E_{network}$, the network entropy $S_{network}$, and the CNT content, by using a machine learning model.

FIG. 5 illustrates changes in network entropy and network energy with respect to changes in CNT content. FIG. 5 may show a contour diagram showing a CNT content using the network energy $E_{network}$ and the network entropy $S_{network}$ as indicators. Referring to FIG. 5, the relationship between the network energy $E_{network}$, the network entropy $S_{network}$, and the CNT content may be identified. It may be seen that a CNT content change may cause a more change to the network entropy $S_{network}$ than to the network energy $E_{network}$.

According to an embodiment, the processor 140 may establish a relational expression (or operation formular), based on the information about the relationship between the network energy $E_{network}$, the network entropy $S_{network}$, the network temperature $T_{network}$, and the CNT content. Specifically, the processor 140 may establish a relational expression (or operation formular) between the network energy $E_{network}$, the network entropy $S_{network}$, the network temperature $T_{network}$, and the CNT content, as in Equation 4.

As described above, the network temperature $T_{network}$ may include a constant preset according to a content of a CNT included in a standard sample, and the network energy $E_{network}$ and the network entropy $S_{network}$ may include variables.

That is, the processor 140 may derive a CNT content by inputting the network temperature $T_{network}$, which is a constant, and the network energy $E_{network}$ and the network entropy $S_{network}$, which are variables, into Equation 4.

[Equation 4]

$$NT_{Conc} = E_{network} - T_{network} * S_{network}$$

Herein, $NT_{Conc}$ may mean a CNT content. Referring to FIG. 6, a correlation (or Pearson correlation coefficient R) of 0.997 may be seen between a CNT content of the standard samples 115 and a value derived by Equation 4.

Measuring CNT content based on Relational Expression

According to an embodiment, the diagnosis apparatus 101 may calculate a content of a CNT included in a random carbon mixture sample having no CNT content information, based on the relational expression between the network energy $E_{network}$, the network entropy $S_{network}$, the network temperature $T_{network}$, and the CNT content, obtained from the standard samples through a series of operations.

Specifically, the diagnosis apparatus 101 may obtain information about a Raman spectrum for a random sample from the Raman spectroscope 103. Thereafter, the diagnosis apparatus 101 may analyze a Raman spectrum to obtain an adjacency matrix and a distance matrix. Thereafter, the diagnosis apparatus 101 may obtain the network energy $E_{network}$ and the network entropy $S_{network}$, based on the obtained adjacency matrix and distance matrix. Last, the diagnosis apparatus 101 may obtain the CNT content by inputting the obtained network energy $E_{network}$ and network entropy $S_{network}$ to the relational expression (or operation formular).

As described above, the diagnosis apparatus 101 may obtain the CNT content from the Raman spectrum. The diagnosis apparatus 101 may also diagnose the state of the battery through the CNT content obtained from the Raman spectrum. Thus, a method, performed by the diagnosis apparatus 101, of a CNT content from a Raman spectrum may be used to predict and diagnose a lifetime of a battery in manufacturing and use (charging/-discharging) of the battery or determining whether to reuse (or recycle) a waste battery.

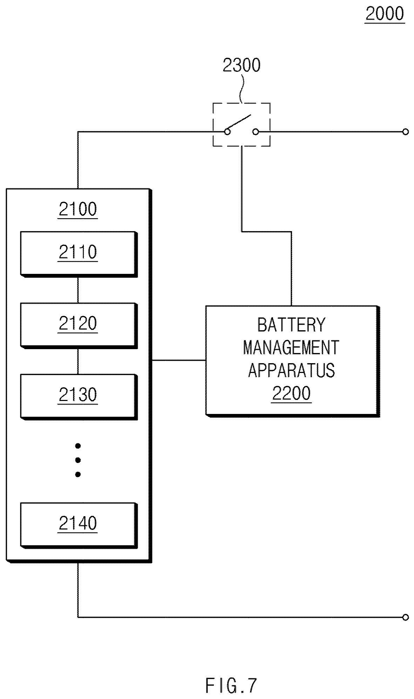

FIG. 7 is a flowchart of an operating method of a diagnosis apparatus according to an embodiment disclosed herein.

Hereinbelow, an operating method of the diagnosis apparatus 101 will be described with reference to FIGS. 1 to 6.

The diagnosis apparatus 101 may be substantially the same as the diagnosis apparatus 101 described with reference to FIGS. 1 to 6, and thus will be briefly described to avoid redundant description.

Referring to FIG. 7, an operating method of a diagnosis apparatus may include operation S101 of obtaining information about a Raman spectrum for a standard sample from a Raman spectroscope, operation S102 of obtaining an intensity of nodes divided by reference wavenumber spacings based on the information about the Raman spectrum, operation S103 of generating a network for the nodes based on intensities of the nodes, operation S104 of establishing a CNT content operational formular based on characteristics of the network, and operation S105 of calculating a CNT content of a sample based on the CNT content operational formular.

In operation S101, the diagnosis apparatus 101 may obtain the Raman spectrum for the standard sample. According to an embodiment, the diagnosis apparatus 101 may obtain the Raman spectrum for the standard sample from the Raman spectroscope 103. Herein, CNT contents of the standard samples may be respectively 0, 25, 33.3, 50, 66,7, and 100 wt%.

In operation S102, the diagnosis apparatus 101 may set an intensity of a unit Raman spectrum. Herein, the intensity of the unit Raman spectrum may be set by an intensity of a start point, an intensity of a mid-point, an intensity of the last point, or an average intensity for each section of a wavenumber range.

In operation S102, the diagnosis apparatus 101 may classify unit Raman spectra as nodes in intensity ranges 311 to 319 for respective reference wavenumber sections, based on intensities of unit Raman spectra.

In operation S103, the diagnosis apparatus 101 may generate a network based on the Raman spectrum. According to an embodiment, the diagnosis apparatus 101 may generate an adjacency matrix and a distance matrix based on unit Raman spectra. Elements of the adjacency matrix may indicate adjacencies between nodes, and elements of the distance matrix may indicate distances between the nodes.

In operation S103, according to an embodiment, the diagnosis apparatus 101 may generate a network of the nodes, based on the adjacency matrix and the distance matrix. Specifically, the diagnosis apparatus 101 may generate a connection structure (a distance and adjacency) between the nodes constituting the network, based on values of the elements of the adjacency matrix and the distance matrix.

In operation S103, according to an embodiment, the diagnosis apparatus 101 may obtain characteristics of the network (e.g., the network energy $E_{network}$, the network entropy $S_{network}$, and the network temperature $T_{network}$), based on the network.

In operation S104, the diagnosis apparatus 101 may establish a CNT content operation formular based on the characteristics of the network. According to an embodiment, the diagnosis apparatus 101 may establish the relational expression (or operation formular), based on the information about the relationship between the network energy $E_{network}$, the network entropy $S_{network}$, and the CNT content.

In operation S105, the diagnosis apparatus 101 may calculate a content of a CNT included in a new sample based on the established relational expression (or operation formular).

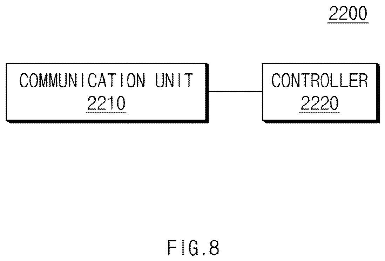

FIG. 8 illustrates a battery pack according to an embodiment disclosed herein.

Referring to FIG. 8, a battery pack 2000 according to an embodiment disclosed herein may include a battery module 2100, a battery management apparatus 2200, and a relay 2300. According to various embodiments, the battery module 2100 may be a battery cell, and in this case, the battery pack 2000 may have a cell-to-pack structure.

Meanwhile, although one battery module 2100 is illustrated in FIG. 8, the battery module 2100 may be configured in plural and the battery pack 2000 may have a stacked structure of a plurality of battery modules, according to an embodiment. The battery module 2100 may include a plurality of battery cells 2110, 2120, 2130, and 2140. Although the plurality of battery cells are illustrated as four in FIG. 8, the present disclosure is not limited thereto, and the battery module 2100 may include n battery cells (n is a natural number equal to or greater than 2). Each of the plurality of battery cells 2110, 2120, 2130, and 2140 may be a cell group or a battery bank in which at least two battery cells are connected in parallel.

The battery module 2100 may supply power to a target device (not shown). To this end, the battery module 2100 may be electrically connected to the target device. Herein, the target device may include an electrical, electronic, or mechanical device that operates by receiving power from the battery pack 2000 including the plurality of battery cells 2110, 2120, 2130, and 2140, and the target device may be, for example, an electric vehicle (EV) or an energy storage system (ESS), but is not limited thereto.

The plurality of battery cells 2110, 2120, 2130, and 2140, each of which is a basic unit of a battery available by charging and discharging electrical energy, may be a lithium ion (Li-ion) battery, an Li-ion polymer battery, a nickel-cadmium (Ni-Cd) battery, a nickel hydrogen (Ni-MH) battery, etc., and are not limited thereto. Meanwhile, although one battery module 2100 is illustrated in FIG. 8, the battery module 2100 may be configured in plural according to an embodiment.

The battery management apparatus (a battery management system (BMS)) 2200 may manage and/or control a state and/or an operation of the battery module 2100. For example, the battery management apparatus 2200 may manage and/or control the states and/or operations of the plurality of battery cells 2110, 2120, 2130, and 2140 included in the battery module 2100. The battery management apparatus 2200 may manage charging and/or discharging of the battery module 2100.

The battery management apparatus 2200 may control an operation of the relay 2300. For example, the battery management apparatus 2200 may short-circuit the relay 2300 to supply power to the target device. The battery management apparatus 2200 may short-circuit the relay 2300 when a charging device is connected to the battery pack 2000.

In addition, the battery management apparatus 2200 may monitor a voltage, a current, a temperature, etc., of the battery module 2100 and/or each of the plurality of battery cells 2110, 2120, 2130, and 2140 included in the battery module 2100. A sensor or various measurement modules for monitoring performed by the battery management apparatus 2200, which are not shown, may be additionally installed in the battery module 2100, a charging/discharging path, any position of the battery module 2100, etc. The battery management apparatus 2200 may calculate a parameter indicating a state of the battery module 2100, e.g., a state of charge (SOC), a state of health (SOH) etc., based on a measurement value such as monitored voltage, current, temperature, etc.

The following operation of the battery management apparatus 2200 may also be performed in the battery management apparatus 2200 or various devices such as a server, a cloud, a charger, a charger/discharger, etc., connected to a vehicle having the battery management apparatus 2200 mounted thereon.

Hereinbelow, the battery management apparatus 2200 will be described with reference to FIGS. 1 to 7.

The battery management apparatus 2200 may be substantially the same as the diagnosis apparatus 101 described with reference to FIGS. 1 to 7, and thus will be briefly described to avoid redundant description.

The battery management apparatus 2200 may be wiredly and/or wirelessly connected to the Raman spectroscope 103.

According to an embodiment, connection between the battery management apparatus 2200 and the Raman spectroscope 103 may be communication connection through a wired and/or wireless network. According to an embodiment, the wired network may be based on an LAN communication or a power-line communication. According to an embodiment, the wireless network may be based on a short-range communication network (e.g., Bluetooth, WiFi, or IrDA) or a remote-range communication network (e.g., a cellular network, a 4G network, a 5G network).

According to another embodiment, the connection between the battery management apparatus 2200 and the Raman spectroscope 103 may be connection using a device-to-device communication scheme (e.g., a bus, a GPIO, an SPI, or an MIPI).

According to an embodiment, the Raman spectroscope 103 may be a device that performs Raman spectroscopy. According to an embodiment, the Raman spectroscope 103 may be a device for identifying a structure, a type, a content, etc., of a molecule included in a sample 115 by using the Raman effect. According to an embodiment, the Raman spectroscope 103 may identify the structure, the type, the content, etc., of the molecule included in the sample 115 based on a principle that energy of laser irradiated to the sample 115 is absorbed by a difference in energy level of electrons of the molecule of the sample 115.

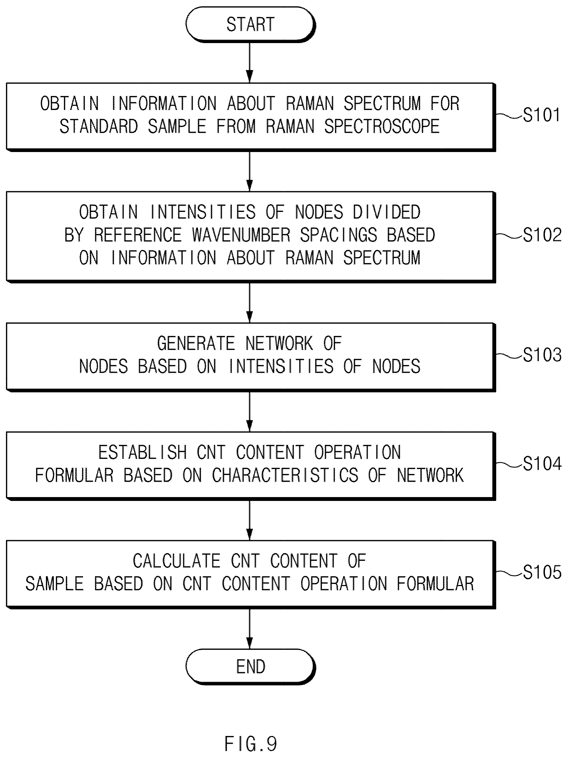

FIG. 9 is a block diagram illustrating a configuration of a battery management apparatus, according to an embodiment disclosed herein.

Referring to FIG. 9, the battery management apparatus 2200 may include a communication unit 2210 and a controller 2220. According to an embodiment, the battery management apparatus 2200 shown in FIG. 9 may further include at least one component (e.g., a display, an input device, or an output device) in addition to components shown in FIG. 9.

According to an embodiment, the communication unit 2210 may establish a wired communication channel and/or a wireless communication channel between the battery management apparatus 2200 and the Raman spectroscope 103, and transmit and receive data to and from the Raman spectroscope 103 through the established communication channel.

The memory (not shown) included in the battery management apparatus 2200 may include a volatile and/or a nonvolatile memory. According to an embodiment, the memory may store data used by at least one component (e.g., the controller 2220) of the battery management apparatus 2200. For example, the data may include a program (or an instruction related thereto), input data, or output data. According to an embodiment, the instruction, when executed by the controller 2220, may cause the battery management apparatus 2200 to perform operations defined by the instruction.

According to an embodiment, the controller 2220 may include a central processing unit, an application processor, a graphic processing unit, a neural processing unit (NPU), an image signal processor, a sensor hub processor, or a communication processor.

According to an embodiment, the controller 2220 may execute software to control at least one other component (e.g., a hardware or software component) of the battery management apparatus 2200 connected to the controller 2220 and may process or compute various data.

Hereinbelow, a detailed description will be made of a method, performed by the battery management apparatus 2200, of quantitatively measuring a content of a CNT included in the sample 115 based on spectrum data obtained from the Raman spectroscope 103.

The Raman spectroscope 103 may obtain Raman spectra 210, 220, 230, 240, 250, and 260 from different standard samples 115. According to an embodiment, the Raman spectroscope 103 may obtain the Raman spectra 210, 220, 230, 240, 250, and 260 in a wavenumber (Raman shift) from 1000 to 1800 cm-1.

The different standard samples 115 may be samples in which the CNT is mixed at different mass ratios (weight percent, wt%). For example, CNT contents of the different standard samples 115 may be respectively 0, 25, 33.3, 50, 66,7, and 100 wt%. For example, the Raman spectra 210, 220, 230, 240, 250, and 260 may be obtained when the CNT contents of the standard samples 115 are 100, 66.7, 50, 33.3, 25, and 0 wt%, respectively.

The Raman spectra 210, 220, 230, 240, 250, and 260 of the different standard samples 115 may have peak values in a D band 201 around a wavelength of 1350 cm-1 and a G band 205 around a wavelength of 1580 cm-1.

The Raman spectroscope 103 may transmit information about the obtained Raman spectrums 210, 220, 230, 240, 250, and 260 of the standard samples 115 having different compositions to the battery management apparatus 2200.

The battery management apparatus 2200 may obtain information about the Raman spectra 210, 220, 230, 240, 250, and 260 from the Raman spectroscope 103 through the communication unit 2210.

The controller 2220 of the battery management apparatus 2200 may analyze the Raman spectra 210, 220, 230, 240, 250, and 260. According to an embodiment, the controller 2220 may divide Raman spectra at reference frequency spacings. The Raman spectrum divided by wavenumber ranges 321 to 330 may be referred to as a unit Raman spectrum.

According to an embodiment, the controller 2220 may set an intensity of the unit Raman spectrum. Herein, the intensity of the unit Raman spectrum may be set by an intensity of a start point, an intensity of a midpoint, an intensity of the last point, or an average intensity for each section of a wavenumber range.

The controller 2220 may classify unit Raman spectra as nodes in intensity ranges 311 to 319 for respective reference wavenumber sections, based on intensities of unit Raman spectra.

It may be seen that a Raman spectrum is subdivided into unit Raman spectra based on a grid of a unit intensity and a unit wavenumber spacing.

According to an embodiment, the controller 2220 may generate an adjacency matrix 400 and a distance matrix 450 based on unit Raman spectra. The elements $A_{11}$, $A_{12}$, ..., $A_{1n}$, $A_{21}$, ..., $A_{nn}$ of the adjacency matrix 400 may indicate adjacencies between the unit spectra $C_1$, $C_2$, ..., $C_n$, and the elements $D_{11}$, $D_{12}$, ..., $D_{1n}$, $D_{21}$, ..., $D_{nn}$ of the distance matrix 450 may indicate distances between the unit spectra $C_1$, $C_2$, ..., $C_n$.

According to an embodiment, unit Raman spectra divided by the wavenumber ranges 321 to 330 may be understood as the unit spectra $C_1$, $C_2$, ..., $C_n$ of the adjacency matrix 400 and the distance matrix 450. For example, a unit Raman spectrum included in the wavenumber range 321 may be understood as a first node $C_1$, a unit Raman spectrum included in the wavenumber range 322 may be understood as a second node $C_2$, and a unit Raman spectrum included in the wavenumber range 323 may be understood as a third node $C_3$.

According to an embodiment, in such a way that unit spectra are set to be adjacent to each other (i.e., their elements have a value of 1) between adjacent intensity ranges and unit spectra are set not to be adjacent to each other (i.e., their elements have a value of 0) between non-adjacent intensity ranges, values of the elements $A_{11}$, $A_{12}$, ..., $A_{1n}$, $A_{21}$, ..., $A_{nn}$ of the adjacency matrix 400 may be assigned. For example, when the node $C_1$ corresponding to the wavenumber range 321 is included in the intensity range 319 and the node $C_2$ corresponding to the wavenumber range 322 is included in the intensity range 318, the elements $A_{12}$ and $A_{21}$ defined by the unit spectra $C_1$ and $C_2$ may have values of 1. In another example, when the node $C_1$ corresponding to the wavenumber range 321 is included in the intensity range 319 and the node $C_3$ corresponding to the wavenumber range 323 is included in the intensity range 316, the elements $A_{12}$ and $A_{13}$ defined by the unit spectra $C_1$ and $C_3$ may have values of 0. A product of assigned 0 or 1 and a weight factor may be used as a value of the elements of the adjacency matrix 400.

According to an embodiment, elements $D_{11}$, $D_{12}$, ..., $D_{1n}$, $D_{21}$, ..., $D_{nn}$ of the distance matrix 450 may be set as distances between the nodes $C_1$, $C_2$, ...,

$C_n$ defined based on a unit intensity section and a unit wavenumber section. For example, a distance between unit spectra included in the same intensity range may be a value of at least 0 according to a difference between unit wavenumber sections. In another example, a distance between nodes included in different intensity ranges may be a value of at least 1 according to a difference between unit wavenumber sections. For example, a distance between a node included in the intensity range 319 and a node included in the intensity range 317 may be 2 in the wavenumber range 321 and the wavenumber range 322, but may be 11 in the wavenumber range 321 and the wavenumber range 324. A product of a distance between two different nodes and a preset weight factor may be used as a value of the elements of the distance matrix 450.

According to an embodiment, the controller 2220 may obtain network characteristics of the nodes $C_1$, $C_2$, ..., $C_n$, based on the adjacency matrix 400 and the distance matrix 450. Herein, the network of the nodes $C_1$, $C_2$, ..., $C_n$ may indicate a connection structure (a distance and adjacency) between the nodes $C_1$, $C_2$, ..., $C_n$. The network characteristics may include a network energy $E_{network}$, a network entropy $S_{network}$, and a network temperature $T_{network}$.

According to an embodiment, the network energy $E_{network}$ may be expressed as a product of the number of nodes included in the network and a function of a distance between the nodes. For example, the network energy $E_{network}$ may be defined by Equation 5 below.

[Equation 5]

$$E_{network,i,j} = a * f(d_{i,j})$$

Herein, a may indicate number information of nodes, and $f(d_{i,j})$ may indicate a function of a distance d between $i^{th}$ and $j^{th}$ nodes. Herein, the function f may be a polynomial function set by a linear or non-linear regression technique. For example, the network energy $E_{network}$ may be a sum of network energies $E_{network,i,j}$ obtained for all nodes.

According to an embodiment, the network entropy $S_{network}$ may be expressed as a product of the number of nodes included in the network and a function of a distance between the nodes. For example, the network entropy $S_{network}$ may be defined by Equation 6 below.

[Equation 6]

$$S_{network} = H * \log H$$

Herein, log may mean a logarithmic function. Herein, H may be defined by Equation 7.

[Equation 7]

$$H = g(1/d_{i,j})$$

$g(1/d_{i,j})$ may indicate a function of a reciprocal of the distance d between the $i^{th}$ and $j^{th}$ nodes. Herein, the function g may be a polynomial function set by a linear or non-linear regression technique. For example, the network entropy $S_{network}$ may be a sum of network entropies $S_{network,i,j}$ obtained for all the nodes.

According to an embodiment, the network temperature $T_{network}$ may be set in advance (e.g., 45.87) according to a content of a CNT included in a standard sample.

According to an embodiment, the controller 2220 may obtain information about a relationship between the network energy $E_{network}$, the network entropy $S_{network}$, and a CNT content. According to an embodiment, the controller 2220 may obtain the information about the relationship between the network energy $E_{network}$, the network entropy $S_{network}$, and the CNT content, based on MLR. According to an embodiment, the information about the relationship may be obtained by a machine learning algorithm such as an SVM, a decision tree, Random Forest, a GBM, artificial neural networks, etc., but the present disclosure is not limited to stated algorithms. According to an embodiment, the controller 2220 may obtain the information about the relationship between the network energy $E_{network}$, the network entropy $S_{network}$, and the CNT content, by using a machine learning model.

The relationship between the network energy $E_{network}$, the network entropy $S_{network}$, and the CNT content may be identified. It may be seen that a CNT content change may cause a more change to the network entropy $S_{network}$ than to the network energy $E_{network}$.

According to an embodiment, the controller 2220 may establish an equation (or operation formular), based on the information about the relationship between the network energy $E_{network}$, the network entropy $S_{network}$, the network temperature $T_{network}$, and the CNT content. Specifically, the controller 2220 may establish a relational expression (or operation formular) between the network energy $E_{network}$, the network entropy $S_{network}$, and the CNT content, as in Equation 8.

As described above, the network temperature Tnetwork may include a constant preset according to a content of a CNT included in a standard sample, and the network energy Enetwork and the network entropy Snetwork may include variables.

That is, the controller 2220 may derive a CNT content by inputting the network temperature $T_{network}$, which is a constant, and the network energy $E_{network}$ and the network entropy $S_{network}$, which are variables, into Equation 8.

[Equation 8]

$$NT_{Conc} = E_{network} - T_{network} * S_{network}$$

Herein, $NT_{Conc}$ may mean a CNT content. A correlation (or Pearson correlation coefficient R) of 0.997 may be seen between a CNT content of the standard samples 115 and a value derived by Equation 8.

Measuring CNT content based on Relational Expression

According to an embodiment, the battery management apparatus 2200 may calculate a content of a CNT included in a random carbon mixture sample having no CNT content information, based on the relational expression between the network energy $E_{network}$, the network entropy $S_{network}$, the network temperature $T_{network}$, and the CNT content, obtained from the standard samples through a series of operations.

Specifically, the battery management apparatus 2200 may obtain information about a Raman spectrum for a random sample from the Raman spectroscope 103. Thereafter, the battery management apparatus 2200 may analyze a Raman spectrum to obtain an adjacency matrix and a distance matrix. Thereafter, the battery management apparatus 2200 may obtain the network energy $E_{network}$ and the network entropy $S_{network}$, based on the obtained adjacency matrix and distance matrix. Last, the battery management apparatus 2200 may obtain the CNT content by inputting the obtained network energy $E_{network}$ and network entropy $S_{network}$ to the relational expression (or operation formular).

As described above, the battery management apparatus 2200 may obtain the CNT content from the Raman spectrum. The battery management apparatus 2200 may also diagnose the state of the battery through the CNT content obtained from the Raman spectrum. Thus, a method, performed by the battery management apparatus 2200, of a CNT content from a Raman spectrum may be used to predict and diagnose a lifetime of a battery in manufacturing and use (charging/discharging) of the battery or determining whether to reuse (or recycle) a waste battery.

**Claims**

1. A diagnosis apparatus comprising:

   a communication circuit configured to obtain, from a Raman spectroscope, information about a Raman spectrum for a mixed sample of different carbon materials comprising a carbon nanotube (CNT); and
   a processor configured to obtain intensities of nodes divided by reference wavenumber spacings of a spectrum, based on the information, generate a network of the nodes based on the intensities, and calculate a CNT content of the sample based on characteristics of the network.

2. The diagnosis apparatus of claim 1, wherein the processor is further configured to calculate the CNT content of the sample based on characteristics of the network comprising a network energy and a network entropy.

3. The diagnosis apparatus of claim 2, wherein the processor is further configured to calculate the network energy and the network entropy and input the network energy and the network entropy to a relational expression to calculate the CNT content.

4. The diagnosis apparatus of claim 3, wherein the processor is further configured to calculate the CNT content based on the relational expression expressed by Equation 1,

   [Equation 1]

   $$NT_{Conc} = E_{network} - T_{network} * S_{network}$$

   wherein $NT_{Conc}$ indicates the CNT content, $E_{network}$ indicates the network energy, $T_{network}$ indicates a network temperature, and $S_{network}$ indicates the network entropy.

5. The diagnosis apparatus of claim 1, wherein the processor is further configured to generate the network defined by an adjacency matrix and a distance matrix of the nodes.

6. The diagnosis apparatus of claim 5, wherein the processor is further configured to set elements of the adjacent matrix indicating adjacencies between the nodes in an intensity range and elements of the distance matrix indicating distances between the nodes divided by the reference wavenumber spacings.

7. An operating method of a diagnosis apparatus, the operating method comprising:

   obtaining, from a Raman spectroscope, information about a Raman spectrum for a sample;
   obtaining intensities of nodes divided by reference wavenumber spacings, based on the information;
   generate a network of the nodes based on the intensities; and
   calculating a carbon nanotube (CNT) content of the sample based on characteristics of the network.

8. The operating method of claim 7, wherein the calculating of the CNT content of the sample based on the characteristics of the network comprises generating the network comprising a network energy and a network entropy.

9. The operating method of claim 8, wherein the calculating of the CNT content of the sample based on the characteristics of the network comprises:

    calculate the network energy and the network entropy; and
    calculating the CNT content by inputting the network energy and the network entropy to a relational expression to calculate the CNT content.

10. The operating method of claim 9, wherein the calculating of the CNT content of the sample based on the characteristics of the network comprises calculating the CNT content based on the relational expression expressed by Equation 1,

    [Equation 1]

    $$NT_{Conc}=E_{network}-T_{network}*S_{network}$$

    wherein $NT_{Conc}$ indicates the CNT content, $E_{network}$ indicates the network energy, $T_{network}$ indicates a network temperature, and $S_{network}$ indicates the network entropy.

11. The operating method of claim 7, wherein the generating of the network of the nodes based on the intensities comprises generating the network defined by an adjacency matrix and a distance matrix of the nodes.

12. The operating method of claim 11, wherein the generating of the network of the nodes based on the intensities comprises setting elements of the adjacent matrix indicating adjacencies between the nodes in an intensity range and elements of the distance matrix indicating distances between the nodes divided by the reference wavenumber spacings.

FIG.1

FIG.2

FIG.3

|  | $C_1$ | $C_2$ | $C_3$ | . . . | $C_n$ |
|---|---|---|---|---|---|
| $C_1$ | $A_{11}$ | $A_{12}$ | $A_{13}$ | . . . | $A_{1n}$ |
| $C_2$ | $A_{21}$ | $A_{22}$ | $A_{23}$ | . . . | $A_{2n}$ |
| $C_3$ | $A_{31}$ | $A_{32}$ | $A_{33}$ | . . . | $A_{3n}$ |
| ⋮ | . . . | . . . | . . . | . . . | . . . |
| $C_n$ | $A_{n1}$ | $A_{n2}$ | $A_{n3}$ | . . . | $A_{nn}$ |

400

|  | $C_1$ | $C_2$ | $C_3$ | . . . | $C_n$ |
|---|---|---|---|---|---|
| $C_1$ | $D_{11}$ | $D_{12}$ | $D_{13}$ | . . . | $D_{1n}$ |
| $C_2$ | $D_{21}$ | $D_{22}$ | $D_{23}$ | . . . | $D_{2n}$ |
| $C_3$ | $D_{31}$ | $D_{32}$ | $D_{33}$ | . . . | $D_{3n}$ |
| ⋮ | . . . | . . . | . . . | . . . | . . . |
| $C_n$ | $A_{n1}$ | $D_{n2}$ | $D_{n3}$ | . . . | $D_{nn}$ |

450

FIG.4

FIG.5

FIG.6

2000

2300

2100

2110

2120

2130

•
•
•

2140

BATTERY
MANAGEMENT
APPARATUS
2200

FIG.7

EP 4 729 928 A1

2200

| COMMUNICATION UNIT 2210 | CONTROLLER 2220 |

FIG.8

20

START

OBTAIN INFORMATION ABOUT RAMAN SPECTRUM FOR STANDARD SAMPLE FROM RAMAN SPECTROSCOPE — S101

OBTAIN INTENSITIES OF NODES DIVIDED BY REFERENCE WAVENUMBER SPACINGS BASED ON INFORMATION ABOUT RAMAN SPECTRUM — S102

GENERATE NETWORK OF NODES BASED ON INTENSITIES OF NODES — S103

ESTABLISH CNT CONTENT OPERATION FORMULAR BASED ON CHARACTERISTICS OF NETWORK — S104

CALCULATE CNT CONTENT OF SAMPLE BASED ON CNT CONTENT OPERATION FORMULAR — S105

END

FIG.9

## INTERNATIONAL SEARCH REPORT

| International application No. |
|---|
| **PCT/KR2024/010389** |

| **A.** | **CLASSIFICATION OF SUBJECT MATTER** |
|---|---|
| | **G01N 21/65**(2006.01)i; **G01J 3/44**(2006.01)i; **H01M 10/42**(2006.01)i |

According to International Patent Classification (IPC) or to both national classification and IPC

| **B.** | **FIELDS SEARCHED** |
|---|---|

Minimum documentation searched (classification system followed by classification symbols)

G01N 21/65(2006.01); G01N 21/00(2006.01); G01N 21/25(2006.01); G01N 21/35(2006.01); G01N 23/223(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 라만(Raman), 분광기(spectrometer), 스펙트럼(spectrum), 함량 (content), 탄소나노튜브(carbon nanotube), 파수(wavenumber)

| **C.** | **DOCUMENTS CONSIDERED TO BE RELEVANT** |
|---|---|

| Category* | Citation of document, with indication, where appropriate, of the relevant passages | Relevant to claim No. |
|---|---|---|
| Y<br>A | YUE et al. Generalized Wavelet Shrinkage of Inline Raman Spectroscopy for Quality Monitoring of Continuous Manufacturing of Carbon Nanotube Buckypaper. IEEE Transactions on Automation Science and Engineering. August 2016, Vol. 14, pp. 1-12.<br>See pages 3, 5-6 and 10. | 1,7<br>2-6,8-12 |
| Y | KR 10-2010-0074600 A (POSCO) 02 July 2010 (2010-07-02)<br>See claim 1. | 1,7 |
| A | JP 10-206321 A (SHIO JIGIYOU CENTER) 07 August 1998 (1998-08-07)<br>See paragraphs [0017]-[0021]. | 1-12 |
| A | JP 2012-068084 A (E & M K.K. et al.) 05 April 2012 (2012-04-05)<br>See paragraphs [0011]-[0025]. | 1-12 |
| A | US 2001-0017195 A1 (TRUNG et al.) 30 August 2001 (2001-08-30)<br>See claims 1-9 and figure 1. | 1-12 |

☐ Further documents are listed in the continuation of Box C.　　☑ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
|---|---|
| **22 October 2024** | **22 October 2024** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

## INTERNATIONAL SEARCH REPORT
### Information on patent family members

International application No.

**PCT/KR2024/010389**

| Patent document cited in search report | | | Publication date (day/month/year) | Patent family member(s) | | | Publication date (day/month/year) |
|---|---|---|---|---|---|---|---|
| KR | 10-2010-0074600 | A | 02 July 2010 | None | | | |
| JP | 10-206321 | A | 07 August 1998 | JP | 3085915 | B2 | 11 September 2000 |
| JP | 2012-068084 | A | 05 April 2012 | JP | 5697388 | B2 | 08 April 2015 |
| | | | | WO | 2012-039130 | A1 | 29 March 2012 |
| US | 2001-0017195 | A1 | 30 August 2001 | AT | E289415 | T1 | 15 March 2005 |
| | | | | AU | 2495501 | A | 09 July 2001 |
| | | | | AU | 773411 | B2 | 27 May 2004 |
| | | | | BR | 0016679 | A | 01 October 2002 |
| | | | | CA | 2392292 | A1 | 05 July 2001 |
| | | | | CA | 2392292 | C | 27 September 2005 |
| | | | | DE | 60018208 | T2 | 12 January 2006 |
| | | | | EP | 1118841 | A2 | 25 July 2001 |
| | | | | EP | 1240511 | A1 | 18 September 2002 |
| | | | | EP | 1240511 | B1 | 16 February 2005 |
| | | | | ES | 2237487 | T3 | 01 August 2005 |
| | | | | JP | 2001-272256 | A | 05 October 2001 |
| | | | | JP | 2003-518622 | A | 10 June 2003 |
| | | | | PT | 1240511 | E | 31 May 2005 |
| | | | | US | 2001-0020394 | A1 | 13 September 2001 |
| | | | | US | 6551451 | B2 | 22 April 2003 |
| | | | | WO | 01-48472 | A1 | 05 July 2001 |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 1020230093391